# EUROPEAN PATENT APPLICATION

(11) **EP 3 485 853 A1**
(43) Date of publication of application: **22.05.2019**
(21) Application number: 16908853.1
(22) Date of filing: 14.07.2016
(51) Int. Cl.: A61F 5/02, A41D 13/05

(54) **LOWER BODY SUPPORT APPARATUS AND LOWER BODY SUPPORT METHOD**

(71) Applicant: Helinx Japan Corp., Tokyo 107-0052 (JP)
(72) Inventor: NISHI, Taketane, Chigasaki-shi Kanagawa 253-0053 (JP)
(74) Representative: Brookes IP
(86) International application number: PCT/JP2016/070880
(87) International publication number: WO 2018/011951

(57) **Abstract**

To provide a practical technique for assisting the lower body of a subject by internally rotating thigh muscles.

A lower body assisting device includes a right leg strap 10R for internally rotate right thigh muscles, a left leg strap 10L for internally rotate left thigh muscles, and a waist/hip strap 20 linking them to each other. Both ends of the waist/hip strap 20 is connected to the back sides of the right and left leg straps 10R and 10L, respectively.

## Description

### Technical field

The present invention relates to techniques for assisting or improving the motion of the lower body of a subject.

### Background art

Human beings are capable of upright bipedal walking and human bodies have evolved adaptations to doing this. However, bipedal walking on the earth, where gravity exists, is inherently unnatural, as evident from the fact that many other mammals exhibit quadrupedal walking. Accordingly, the human bodies continually ensure a heavy burden, even in daily life.

Examples are the thigh muscles. Approximately 70% of the thigh muscles are lateral rotators that externally rotate the femur and approximately 30% are medial rotators that internally rotate the femur. Since the lateral rotators constitute most of the thigh muscle complex, the thigh muscles tend toward automatic external rotation in daily life.

As used herein, the lateral rotation of the thigh muscles about the femur (specifically, the "lateral rotation" means that the muscles of the front of the thigh move outward) is referred to as "external rotation" of the thigh muscles. Likewise, as used herein, the medial rotation of the thigh muscles about the femur (specifically, the "medial rotation" means that the muscles of the front of the thigh move inward) is referred to as "internal rotation" of the thigh muscles.

Although external rotation of the thigh muscles is not always dangerous, excess external rotation may induce health problems such as pelvic opening causing lower back pain, bowed legs, difficulties in moving the legs forward during walking, and difficulties in sit-to-stand movement because of the loss of thigh strength during the sit-to-stand movement. In particular, during walking, the internal rotators should play a major role, and walking can be difficult when the external rotators become dominant.

Such problems sometimes occur at a young but become more remarkable with increasing age.

### Summary

### Problems to be solved

It has been pointed out that the external rotation of the thigh muscles can cause problems as described above, and it has also been pointed out that the elimination of the external rotation of the thigh muscles can potentially resolve such issues.

However, at present, there is no realistic solution by which this can be achieved. Exercise and stretching of the muscles of a subject or massaging the body of the subject may assist in inducing the internal rotation of the externally-rotated thigh muscles closer to the ideal condition.

However, the resultant effect is extremely temporary and it is impossible to say that the resultant effect is sufficient.

An object of the present invention is to provide a practical technique for assisting the lower body of a subject by internally rotating thigh muscles.

### Means to solve the problems

In order to solve the aforementioned problems, the present inventor proposes the following inventions.

The invention is a lower body assisting device including: a right leg strap being a strip-shaped substance for exerting, on the right thigh of a subject, a force making the right thigh muscles rotate internally by wrapping the right leg strap around the right thigh at a predetermined compression pressure in use; and a left leg strap being a strip-shaped substance for exerting, on the left thigh of the subject, a force making the left thigh muscles rotate internally by wrapping the left leg strap around the left thigh at a predetermined compression pressure in use.

Such lower body assisting device includes the right and left leg straps each of which is a strip-shaped substance. The right leg strap exerts, on the right thigh muscles, a force making the right thigh muscles rotate internally when wrapped around the right leg of the subject. The left leg strap exerts, on the left thigh muscles, a force making the left thigh muscles rotate internally when wrapped around the left leg of the subject. For example, the right leg strap makes the thigh muscles internally rotate by being wrapped around the right thigh in a direction of internal rotation of the thigh muscles, or by being shifted in a direction of internal rotation of the thigh muscles after the right leg strap is secured around the right thigh with a predetermined compression pressure. The same principle applies to the left leg strap.

The right and left leg straps each of which is the strip-shaped substance can be worn on the legs for a relatively long period of time (e.g., ≥20 minutes, or for 2-3 hours in daily life). Consequently, the internal rotation of the thigh muscles of the subject improves better than when subject performed exercises or stretching or when subject was received massage. Moreover, the effect of such internal rotation can be sustained for a longer period.

Such internal rotation mentioned above on the thigh muscles may alleviate the lower back pain or the issues with bowed legs. The state of the thigh muscles forced to internally rotate by the right and left leg straps resembles the state in which one leg is advanced one step while walking. Thus, it is easier to move the legs forward during walking, more specifically, when moving the legs forward in a step-by-step movement. In contrast, each of the right and left leg straps exerts a force on the thigh muscles, particularly, on the hamstrings of the back of the thighs against the motion when the leg moves backward in the step-by-step movement or when the retracted leg kicks the ground in a backward direction. Consequently, although the subject can walk naturally, the resistance thereof unintentionally produces a state similar to that achieved during thigh muscle strength training, which improves the strength of the thigh muscles. The state of the thigh muscles forced to internally rotate by the right and left leg straps also resembles the state of the muscles during the sit-to-stand movement. Thereby, it becomes easier for a sitting subject wearing the right and leg straps to stand up.

The benefits for the subject wearing the right and left straps provided by the straps in assisting the walking or standing-up movement from a sitting position on a young subject may be less evident because of its lower necessity. However, in an old subject such as a person well on in years or a subject with comparatively imperfect thigh muscles for some reasons, the effects of the right and left leg straps in assisting the walking or standing-up movement from a sitting position are of more significance.

The lower body assisting device may include an anti-rotation member.

The anti-rotation member is for preventing the right leg strap wrapped around the right thigh from rotating in a direction of external rotation of the muscles of the right thigh and preventing the left leg strap wrapped around the left thigh from rotating in a direction of external rotation of the muscles of the left thigh, the anti-rotation member being secured to the body of the subject. With such anti-rotation member, the right and left leg straps of the lower body assisting device are less likely to rotate in a direction of external rotation of the muscles of the respective right and left thigh even when the subject performs exercise such as walking with the straps worn on his/her legs.

The anti-rotation member may be any member as described above. For example, it may either be separate members, i.e., a first member for preventing the right leg strap wrapped around the right thigh from rotating in a direction of external rotation of the right thigh muscles and a second member for preventing the left leg strap wrapped around the left thigh from rotating in a direction of the external rotation of the left thigh muscles or be made of a unitary member. Although the construction of the anti-rotation member can appropriately be determined, it may be in the form of a strip-shaped substance. With the anti-rotation member in the form of the strip-shaped substance, the lower body assisting device can be worn for a relatively long period of time. Whether in the form of the strip-shaped substance or not, the anti-rotation member may be made of a material that stretches lengthwise.

The anti-rotation member may be configured to link the right and left leg straps to each other. For example, when the right and left leg straps are linked to each other on a front side of the body of the subject, the front portions of the right and left leg straps pull against each other. As a result, forces in directions of internal rotation of the right and left thigh muscles are naturally exerted on the respective leg straps.

The anti-rotation member may be a strip-shaped substance having a length that allows the anti-rotation member to connect the back of the right leg strap worn on the right leg of the subject and the back of the left leg strap worn on the left leg of the subject with each other along a path: from the back of the right leg strap to the left ilium of the subject, going around the outside of the right leg strap and along the front side of the body of the subject; from the left ilium, folding back in toward the right ilium of the subject, going along the back side of the body of the subject; and from the right ilium to the back of the left leg, going along the front side of the body of the subject and around the outside of the left leg strap, or vise versa. Such anti-rotation member exerts, on the right and left leg straps, forces in directions of internal rotation of the respective right and left thigh muscles. Further, in addition to this, when the anti-rotation member is guided along the aforementioned path, the anti-rotation member passes the right greater trochanter (or its vicinity) in the path from the back of the right leg strap to the left ilium of the subject, going around the outside of the right leg strap and the front side of the body of the subject; and the anti-rotation member passes the right greater trochanter (or its vicinity) in the path from the right ilium to the back of the left leg strap of the subject, going along the front side of the body of the subject and around the outside of the left leg strap. With such anti-rotation member, the right and left greater trochanters (or their vicinities; the same applies to below) can be pressed from the body surface toward the midline of the body. This suppresses, for example, the lateral slide of the greater trochanters while walking and thereby further stabilizes walking.

The anti-rotation member may be a separate unit from the right and left leg straps. For example, the anti-rotation member may be configured to be attached to and removed from the right and left leg straps. By constructing the anti-rotation member as the separate unit from the right and left leg straps, it becomes easier to change the width or stretchability of the right and left leg straps. Furthermore, it becomes possible to combine an anti-rotation member of one's choice from among anti-rotation members of different width, stretchability, length, and other attributes to the right and left leg straps. The availability of different options in the anti-rotation members according to, for example, the body shape and physical strength is beneficial. Alternatively, the anti-rotation member may be integrally formed with the right and left leg straps, rather than being attachable in a removable manner. For example, the right leg strap, the anti-rotation member, and the left leg strap may be linked in this order and compose a unitary strip-shaped substance. The anti-rotation member united with the right and left leg straps has a simple structure and is easier to handle.

A substantial half of the longitudinal length (e.g., 40%-50% of the length) of a part of the right and left leg straps may be less easily stretchable, the longitudinal length of the part of each of the right leg strap and the left leg strap corresponding to a circumference of the respective right and left thighs. As used herein, the term "less easily stretchable" indicates that a rate of elongation {[(length after elongation - original length)/original length] × 100(%)} is ≤5% when measured by securing the strip-shaped substance across its entire width at predetermined positions, with 30-cm intervals and exerting a load of 2 kg downward at the center between the secured positions of the strip-shaped substance. The aforementioned method of measuring the rate of elongation is referred to as Measurement Method A in this application. The portions of the right and left leg straps which are less easily stretchable are the portions that rest on the back of the respective right and left leg straps when worn on the right and left thighs of the subject. This serves to achieve efficient internal rotation of the muscles of the hamstrings.

As described above, the right and left leg straps are used for internally rotating the thigh muscles. For such internal rotation of the thigh muscles, they should follow the right and left leg straps. If the right and left leg straps have extensive stretchability, the muscles that are forced to internally rotate starts rotating externally; this lets the right and left leg straps to shift the original position. With the right and left leg straps made of a less stretchable material as described above, it is possible to prevent the right or left leg strap from shifting on the thigh muscles, and in turn, prevent the thigh muscles that have internally rotated from rotating externally.

Nevertheless, the fitting to the thigh is improved when the right and left leg straps can be stretched to a certain degree as a whole. This stretchability can be ensured by increasing the stretchability of lengthwise portions of the respective right and left leg straps other than the aforementioned less-stretchable portion, relative to the less-stretchable portion (e.g., the rate of elongation is at least ≥10% as measured using the aforementioned Measurement Method A). As used herein, the term "have stretchability" with respect to the strip-shaped substance indicates an elongation of ≥10% as measured using the Measurement Method A.

Upper and lower edges of each of the right leg strap and the left leg strap, both of which are in the form strip-shaped substances, in use may be curved upwardly and the upper edge may be longer than the lower edge. With this, the right leg strap and the left leg strap fit on the respective legs of the subject when worn thereon.

The lower body assisting device according to the present application may include an upper body strap, the upper body strap being a strip-shaped substance having a length that allows the upper body strap to connect the outside of the right leg strap worn on the right leg of the subject and the outside of the left leg strap worn on the left leg of the subject with each other along a path: from the outside of the right leg strap to the top of the left shoulder of the subject along the back side of the body of the subject; from the top of the left shoulder to the left underarm of the subject along the front side of the body of the subject; from the left underarm to the right underarm of the subject along the back side of the body of the subject; from the right underarm to the top of the right shoulder of the subject along the front side of the body of the subject; and from the top of the right shoulder to the outside of the left leg strap along the back side of the body of the subject, or vise versa, wherein both ends of the upper body strap can be attached to and removed from the respective outsides of the right leg strap and the left leg strap.

Such additional upper body strap helps retract the shoulders of the subject backward and thereby allow him/her to assume an upright posture with the chest puffed out. Against the swing and rotation of the body of the subject while walking, the upper body strap exerts a force to return the swinging or rotating body to its original position, providing effects in reducing the swing oscillation and rotation while assisting walking.

In this case, the upper body strap may be stretchable lengthwise. This enhances the aforementioned benefits of the upper body strap.

As described above, the anti-rotation member may be a strip-shaped substance having a length that allows the anti-rotation member to connect the back of the right leg strap worn on the right leg of the subject and the back of the left leg strap worn on the left leg of the subject with each other along a path: from the back of the right leg strap to the left ilium of the subject, going around the outside of the right leg strap and along the front side of the body of the subject; from the left ilium, folding back in toward the right ilium of the subject, going along the back side of the body of the subject; and from the right ilium to the back of the left leg, going along the front side of the body of the subject and around the outside of the left leg strap, or vise versa. When the lower body assisting device having such anti-rotation member includes the upper body strap as described above, the lower body assisting device may further include a vertical connection member which is configured so as to connect a substantial center of the portion of the upper body strap running from the left underarm to the right underarm along the back side of the body of the subject and a substantial center of the portion of the anti-rotation member running from the left ilium to the right ilium along the back side of the body of the subject. With such vertical connection member, a substantial center portion of the upper body strap running from the left underarm to the right underarm along the back side of the body of the subject is pulled downward, which further extends the spine of the subject, further correcting his/her posture. In this case, the vertical connection member may be a strip-shaped substance. In this case, the vertical connection member may be stretchable lengthwise.

Furthermore, the present inventor also proposes the following lower body assisting method as an aspect of the present invention to achieve the object of the present application.

The lower body assisting method includes, for example, the steps of: wrapping a right leg strap, being a strip-shaped substance, around the right thigh of a subject in such a manner that the right leg strap exerts, on the right thigh, a force making the right thigh muscles rotate internally; and wrapping a left leg strap, being a strip-shaped substance, around the left thigh of the subject in such a manner that the left leg strap exerts, on the left thigh, a force making the left thigh muscles rotate internally. This ensures internal rotation of both of the right and left thigh muscles.

### Brief description of the drawings

Fig. 1 is a view of a left leg strap included in a lower body assisting device according to a first embodiment (a) viewed from the front side, (b) viewed from the reverse side, and (c) viewed sideways;
Fig. 2 is a view of a waist/hip strap included in the lower body assisting device according to the first embodiment (a) viewed from the front side, (b) viewed from the reverse side, and (c) viewed sideways;
Fig. 3 is a view of an upper body strap included in the lower body assisting device according to the first embodiment (a) viewed from the front side, and (b) viewed from the reverse side;
Fig. 4 is a perspective view for use in describing how a right leg strap included in the lower body assisting device according to the first embodiment is put on the right thigh of a subject;
Fig. 5 is a perspective view of right and left leg straps included in the lower body assisting device according to the first embodiment in a state where they are worn around the right and left thighs of a subject;
Fig. 6 is a view of a subject seen from the front, for use in describing how to secure both ends of the waist/hip strap to the right and left leg straps worn around the right and left thighs of the subject, respectively;
Fig. 7 is a front view of the waist/hip strap in a state where the ends thereof are secured to the right and left leg straps worn around the respective thighs of a subject;
Fig. 8 is a side view of the waist/hip strap in the state where the ends thereof are secured to the right and left leg straps worn around the respective thighs of the subject;
Fig. 9 is a back view of the waist/hip strap in the state where the ends thereof are secured to the right and left leg straps worn around the respective thighs of the subject;
Fig. 10 is a view of a subject seen from the front, for use in describing how to secure the upper body strap to the waist/hip strap;
Fig. 11 is a view of a subject seen from the back, illustrating a process of securing the upper body strap to the waist/hip strap;
Fig. 12 is a front view of a subject wearing all of the right leg strap, the left leg strap, the waist/hip strap, and the upper body strap according to the first embodiment of this application;
Fig. 13 is a side view of the subject wearing all of the right leg strap, the left leg strap, the waist/hip strap, and the upper body strap according to the first embodiment of this application;
Fig. 14 is a back view of the subject wearing all of the right leg strap, the left leg strap, the waist/hip strap, and the upper body strap according to the first embodiment of this application;
Fig. 15 is a view of a left leg strap included in a lower body assisting device according to a modified version 1 (a) viewed from the front side, and (b) viewed from the reverse side;
Fig. 16 is a view of a left leg strap included in a lower body assisting device according to a modified version 2 (a) viewed from the front side, (b) viewed from the reverse side, and (c) viewed sideways;
Fig. 17 is a perspective view for use in describing how the left leg strap included in the lower body assisting device according to the modified version 2 is put on the left thigh of a subject;
Fig. 18 is a perspective view for use in describing the left leg strap included in the lower body assisting device according to the modified version 2 in a state where it is worn around the left thigh of a subject;
Fig. 19 is a view of a lower body assisting device according to a second embodiment (a) viewed from the front side, (b) viewed from the reverse side, and (c) viewed sideways; and
Fig. 20 is a view of a subject seen from the front, for use in describing how to put the lower body assisting device according to the second embodiment on the body of a subject.

### Detailed Description

Hereinafter, preferable first and second embodiments of the present invention are described in detail with reference to the drawings.

It is to be noted that corresponding parts are identified by the same reference numerals throughout the embodiments and modified versions thereof and similar descriptions will not again be made, when appropriate.

### <<First embodiment>>

A lower body assisting device according to a first embodiment is mainly made up of a right leg strap, a left leg strap, a waist/hip strap, and an upper body strap. The waist/hip strap is an example of the anti-rotation member in the present application.

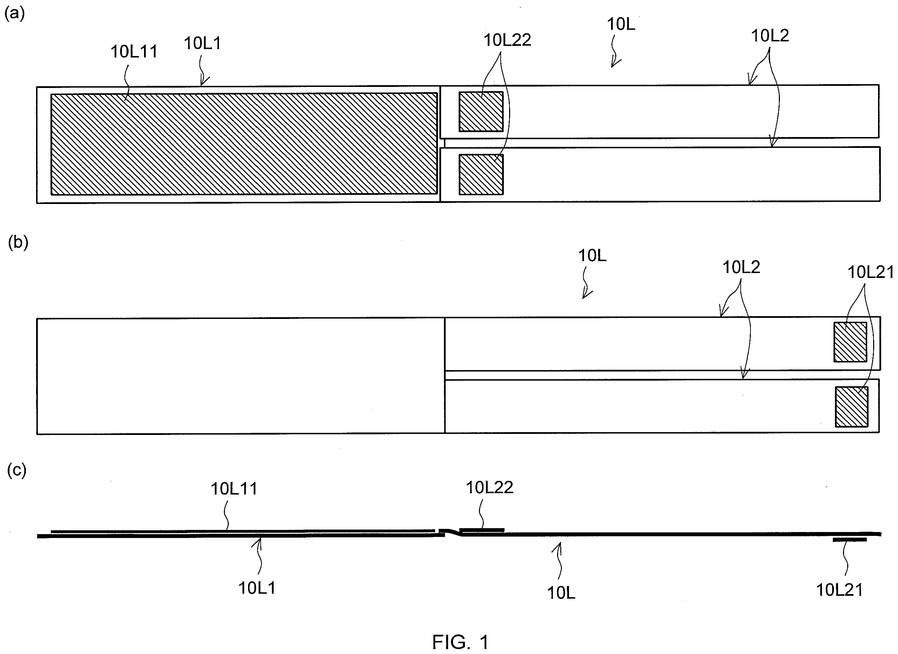

Fig. 1 shows an example of a left leg strap 10L of this application.

The left leg strap 10L is used with being wrapped around and secured to the left thigh of the subject, as described later. The left leg strap 10L compresses the thigh muscles of the subject and exerts a force on the muscles in a direction of internal rotation. The same applies to a right leg strap 10R. The right leg strap 10R is used with being wrapped around and secured to the right thigh of the subject. The right leg strap 10R compresses the thigh muscles of the subject and exerts a force on the muscles in a direction of internal rotation.

The right leg strap is in mirror symmetry to the left leg straps 10L shown in Fig. 1. Note that the left leg strap 10L shown in Fig. 1 is vertically symmetric; thus the horizontal flip of the left leg strap 10L in Fig. 1 is equivalent to the right leg strap.

Hereinafter, the construction of the left leg strap 10L is described as an example of the leg straps. The same reference numerals as those used in the following description of the left leg strap 10L, but with the suffix R instead of L, indicate parts of the right leg strap identical to those of the left one. For example, the reference numeral for the right leg strap in the following description is 10R.

The entire left leg strap 10L is formed as a strip-shaped substance. The left leg strap 10L includes, but not limited to, a rectangular main body unit 10L1 and two elongated securing units 10L2 extending to the right of the main body unit, each of which has, but not limited to, a rectangular shape in this embodiment.

The sum of the lengthwise (i.e., in the horizontal direction in Fig. 1) dimension of the main body unit 10L1 and that of the securing unit 10L2 should be longer than at least the circumference of the thigh of the subject on which the left leg strap 10L is worn (if possible, it is preferable that the total length is longer by at least 10 cm than the circumference of the thigh of the subject). In this embodiment, assuming that the length of the circumference of the thigh of the subject is approximately 35-50 cm, the sum of the lengthwise dimension of the main body unit 10L1 and that of the securing unit 10L2 is approximately 60 cm.

The main body unit 10L1 is made of cloth. The main body unit 10L1 is almost rectangular, and has a lengthwise dimension of, but not limited to, about 25 cm. This length is determined in consideration that the outer circumference of the thigh of the subject who is expected to put on the left leg strap 10L is approximately 50-60 cm. It is preferable that the length of the main body unit 10L1 is almost half the outer circumference of the thigh of the subject who is expected to put on the left leg strap 10L, more specifically, 40-50% of the outer circumference of the thigh. Further, the width (in the vertical dimension in Fig. 1) of the main body unit 10L1 is, but not limited to, about 10 cm. It is preferable that the width of the main body unit 10L1 is, but not limited to, approximately 5-15cm. With a width of ≤5 cm, the subject wearing the left leg strap 10L and the right leg strap could feel a pain. Conversely, a width of ≥15 cm prevents the subject from smoothly moving, e.g., walking.

To the front side of the main body unit 10L1, a main body securing member 10L11 is attached for achieving, in cooperation with a securing unit securing member that is described later, the purpose of securing the left leg strap 10L to the left thigh in a removable manner with the left leg strap 10L wrapped around the left thigh of the subject. The term "front side" of the left leg strap 10L indicates the surface exposed outside when the left leg strap 10L is secured to the body of the subject. The opposite is the reverse side. The definitions of the front and reverse sides also apply to other components such as the right leg strap, the waist/hip strap, and the upper body strap.

The main body securing member 10L11 is a hook-and-loop fastener and more particularly a Velcro-brand tape in this embodiment, but not limited thereto. However, the main body securing member 10L11 and the securing unit securing member may be any combinations as long as they can removably put the left leg strap 10L on the left thigh of the subject in cooperation with each other and may be replaced with, for example, a button and a button hole, two prong snap buttons, ring snap fasteners, jeans buttons and other metal interlocking discs. In this embodiment, the main body securing member 10L11 which is the hook-and-loop fastener as described above is secured to the main body unit 10L1 with a known or widely known suitable method such as adhesive or sewing.

The main body unit 10L1 is less easily stretchable lengthwise (i.e., in the horizontal direction in Fig. 1). As used herein, "less easily stretchable " indicates that, when the rate of elongation of a material similar to the main body unit 10L1 is measured using the aforementioned Measurement Method A, the rate of elongation is ≤5%. To achieve this, for example, a piece of cloth that is less easily stretchable can be used for the main body unit 10L1; or alternatively, a hook-and-loop fastener that is less easily stretchable is used and the almost entire surface of the main body unit 10L1 can be covered with the main body securing member 10L11 in the form of the hook-and-loop fastener. Velcro-brand tapes, commercially available hook-and-loop fasteners, are typically less easily stretchable in all directions. By using this as the main body securing member 10L11 and securing it to the main body unit 10L1 in such a manner that it covers the almost entire surface of the main body unit 10L1, the entire main body unit 10L1 can be made less easily stretchable lengthwise.

Each of the securing units 10L2 has an elongated rectangular shape as described above and is made of cloth. The two securing units 10L2 in the left leg strap 10L shown in Fig. 1 are exactly the same in their construction. The securing units 10L2 may be made as a single piece having the same width as the main body unit 10L1. The securing units 10L2 are secured to the main body unit 10L1 with a known or widely known suitable method such as sewing.

The width of each securing unit 10L2 is, but not limited to, approximately half the width of the main body unit 10L1. The lengthwise dimension of each securing unit 10L2 in this embodiment is, but not limited to, longer than the lengthwise dimension of the main body unit 10L1. More particularly, this length is about 35 cm.

On the reverse sides of the cloth composing the securing units 10L2, at their distal ends, securing unit securing members 10L21 are attached to the respective securing units. Each securing unit securing member 10L21 in this embodiment is a hook-and-loop fastener, and is capable of being attached to and detached from the main body securing member 10L11. The securing unit securing member 10L21 is secured to the securing unit 10L2 with a known or widely known suitable method such as sewing.

On the front sides of the securing units 10L2, in the vicinity of the proximal ends thereof, hook-and-loop fasteners, more specifically, strap front securing members 10L22 each made of a Velcro tape are attached to the respective securing units. Each strap front securing member 10L22 is, as described later, for allowing it to be secured in a removable manner to the waist/hip strap, more specifically, a waist/hip strap reverse left securing member that is also in the form of a hook-and-loop fastener provided on the waist/hip strap. In that sense, the strap front securing members 10L22 and the waist/hip strap reverse left securing members are not necessarily hook-and-loop fasteners. As in the case of the main body securing member 10L11 and the securing unit securing members 10L21, they may be replaced with, for example, a button and a button hole or metal interlocking discs. It is noted that, in the following description, a hook-and-loop fastener indicated as a way to achieve removable attachment can also be replaced with, for example, a button and a button hole or metal interlocking discs.

The securing units 10L2 are made of stretchable material that can be stretched lengthwise. With this, the left leg strap 10L is stretchable lengthwise. This helps in achieving better fitting of the left leg strap 10L onto the circumference of the thigh when the left leg strap 10L is secured to the left thigh of the subject. The stretchability of each securing unit 10L2 can be, for example, about 10-40%, preferably about 20% ± 5% in the rate of elongation when measured using the aforementioned Measurement Method A. The securing unit securing members 10L21 and the strap front securing members 10L22, which are hook-and-loop fasteners in this embodiment, provided on the securing units 10L2 occupy only a very small area of the respective securing members 10L2 at their lengthwise distal ends, as shown in Fig. 1(c). Accordingly, they do not affect the stretchability of the entire securing unit 10L2.

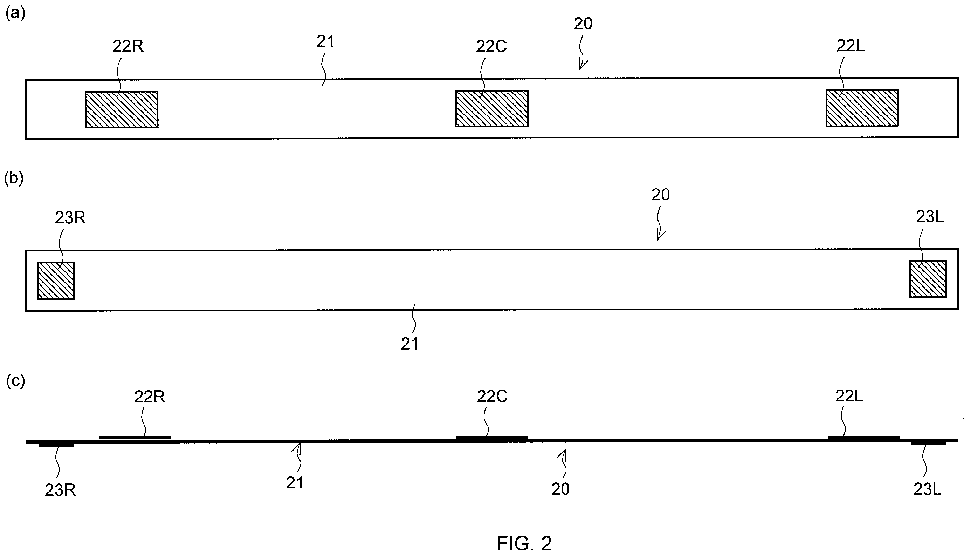

Next, the construction of the waist/hip strap is described with reference to Fig. 2.

As described above, the waist/hip strap 20 corresponds to the anti-rotation member of the present application and is linked to the right and left leg straps 10R and 10L worn around the respective thighs of the subject in a manner describe later. The waist/hip strap 20 exerts, on the right leg strap 10R connected thereto, a force making the right leg strap 10R rotate in a direction of internal rotation, and exerts, on the left leg strap 10L connected thereto, a force making the left leg strap 10L rotate in a direction of internal rotation.

The waist/hip strap 20 in this embodiment is, but not limited to, a strip-shaped substance. The waist/hip strap 20 has a waist/hip strap main body 21. The waist/hip strap main body 21 in this embodiment has, but not limited to, an elongated rectangular shape with the same width along the entire length thereof, as shown in Fig. 2. It is preferable that the length of the waist/hip strap main body 21 is almost equal to one and a half times the waist circumference of the subject. The length in this embodiment is, but not limited to, about 150 cm. It is preferable that the width thereof is about 5-15 cm. The reason for this is the same as the reason the width of the left leg strap 10L should be 5-15 cm. The width of the waist/hip strap main body 21 in this embodiment is, but not limited to, about 5 cm. The waist/hip strap main body 21 is made of cloth that is stretchable lengthwise. The stretchability of the waist/hip strap main body 21 can be about 20-40%, preferably about 30% ± 5% in the rate of elongation when measured using the aforementioned Measurement Method A.

On the front side of the waist/hip strap main body 21 as a strip-shaped substance, at positions near the ends thereof, a waist/hip strap front right securing member 22R and a waist/hip strap front left securing member 22L both of which are hook-and-loop fasteners are secured thereto for the removable attachment to the upper body strap described later, more specifically, an upper body strap right securing member and an upper body strap left securing member of the upper body strap both of which are hook-and-loop fasteners. Their attachment to the waist/hip strap main body 21 can be made using a known or widely known technique described above. Further, on the front side of the waist/hip strap main body 21 as a strip-shaped substance, at a position near the center along the length thereof, a waist/hip strap front center securing member 22C in the form of a hook-and-loop fastener is secured to the waist/hip strap main body 21 for the removable attachment to the upper body strap described later, more specifically, an upper body strap center securing member of the upper body strap in the form of a hook-and-loop fastener. The attachment of the waist/hip strap front center securing member 22C to the waist/hip strap main body 21 can be made using a known or widely known technique described above.

On the reverse side of portions near both ends of the waist/hip strap main body 21, at positions near the ends thereof, a waist/hip strap reverse right securing member 23R for the removable attachment to a strap front securing member 10R22 of the right leg strap 10R and a waist/hip strap reverse left securing member 23L for the removable attachment to the strap front securing member 10L22 of the left leg strap 10L are provided. They are all hook-and-loop fasteners and are secured to the waist/hip strap main body 21 using a known or widely known technique described above.

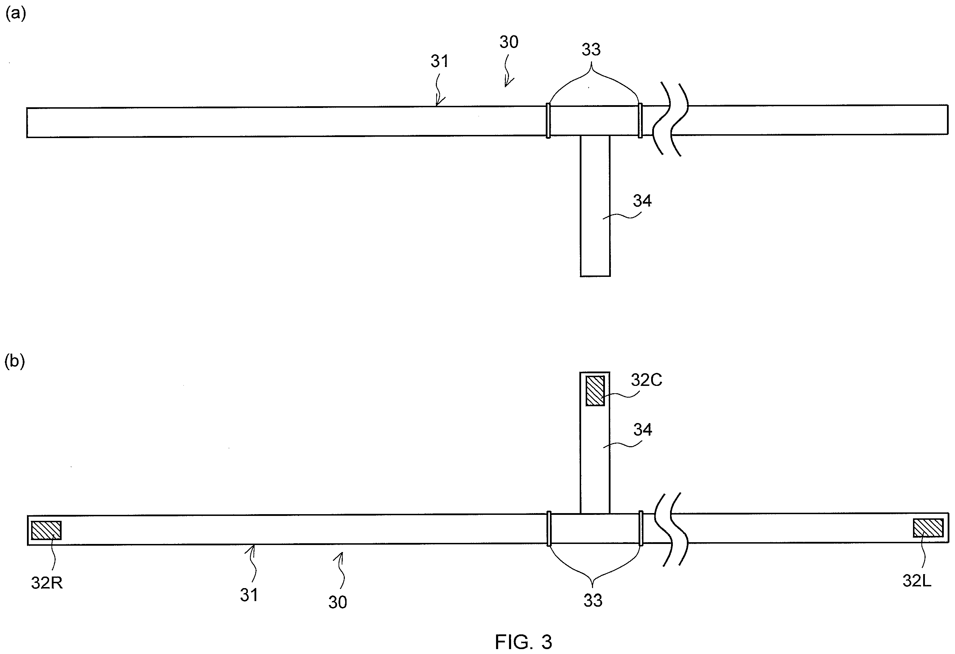

Next, the upper body strap is described with reference to Fig. 3.

The upper body strap 30 is for allowing the subject to assume an upright posture with the chest puffed out and preventing swing oscillation of the subject's body. The upper body strap 30 is connected to the right leg strap 10R and the left leg strap 10L worn around the respective thighs of the subject in a manner described later, and connected to the waist/hip strap worn around the waist of the subject in a manner described later.

The upper body strap 30 in this embodiment is, but not limited to, a strip-shaped substance. The upper body strap 30 has an upper body strap main body 31. The upper body strap main body 31 has, as shown in Fig. 3, a considerably long rectangular shape with the same width along the entire length thereof. It is preferable that the length of the upper body strap main body 31 is such that the length is sufficient for the use of the upper body strap described later. Further, as will be described later, the upper body strap main body 31 can be adjusted in its length. Broadly speaking, the length of the upper body strap main body 31 is determined between 135 cm and 200 cm, though depending on the frame of the subject who puts it on. The upper body strap 30 in this embodiment can be, but not limited to, adjusted in its length within this range. It is preferable that the width thereof is about 5-15 cm. The reason for this is the same as the reason the width of the left leg strap 10L should be 5-15 cm. The width of the upper body strap main body 31 in this embodiment is, but not limited to, about 5 cm. The upper body strap main body 31 is made of cloth that is stretchable lengthwise. The stretchability of the upper body strap main body 31 can be about 30-50%, preferably about 40% ± 5% in the rate of elongation when measured using the aforementioned Measurement Method A.

On the reverse side of the upper body strap main body 31 which is a strip-shaped substance, at positions near the ends thereof, an upper body strap right securing member 32R and an upper body strap left securing member 32L both of which are hook-and-loop fasteners are attached to the upper body strap main body 31 for removable attachment to the waist/hip strap front right securing member 22R and the waist/hip strap front left securing member 22L both of which are hook-and-loop fastener. How to secure them to the upper body strap main body 31 is according to a known or widely known technique described above.

At a position near the center of the length of the upper body strap main body 31, length adjustment members 33 are provided for adjusting the length of the upper body strap main body 31. The length adjustment members 33 can be achieved using a known or widely known technique, which are, but not limited to, two buckles in this embodiment. By adjusting the distance between these buckles, the length of the upper body strap main body 31 running between them in both directions can be adjusted, and in turn, the total length of the upper body strap main body 31 can be adjusted.

At a portion of the upper body strap main body 31 between the two buckles that constitute the length adjustment members 33, a connection band 34 is provided which extends downward in use of the upper body strap 30. The connection band 33 is a strip-shaped substance and has stretchability. Its width and stretchability are according to the upper body strap main body 31. The connection band 34 extends downward from the center between the buckles even when the distance between the buckles constituting the length adjustment members 34. On the reverse side of the connection band 34 at its lower end, an upper body strap center securing member 32C which is a hook-and-loop fastener is provided for the removable attachment to the waist/hip strap front center securing member 22C which is the hook-and-loop fastener on the waist/hip strap 20. The attachment of the upper body strap center securing member 32C to the upper body strap main body 31 is also according to a known or widely known technique described above.

Next, a method of using the lower body assisting device including the right leg straps 10R, the left leg strap 10L, the waist/hip strap 20, and the upper body strap 30 as described above and effects thereof are described.

First, the right leg strap 10R and the left leg strap 10L are secured to the respective thighs of the subject.

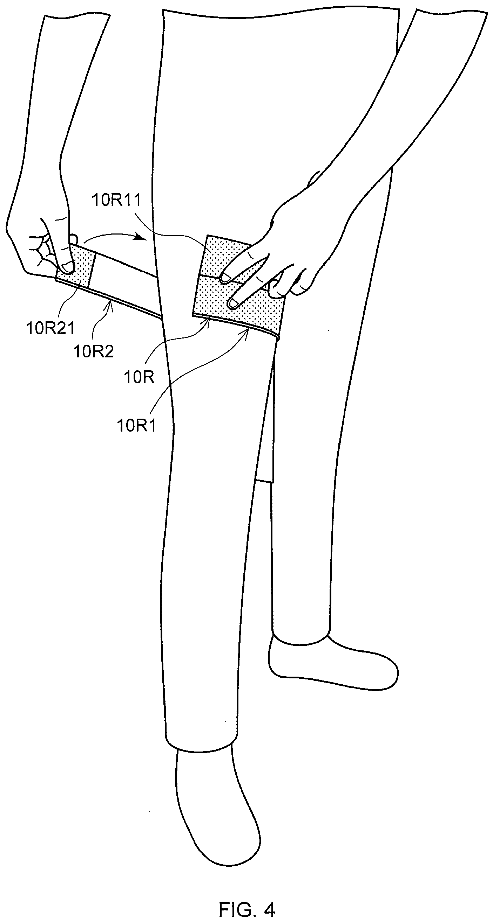

A process of securing the right leg strap 10R to the right thigh of the subject, more specifically, to the thigh at or near the top thereof is shown in Fig. 4. To secure the right leg strap 10R to the right thigh of the subject, the subject (in this embodiment, the description is made assuming that the subject puts on the lower body assisting device by himself/herself but it is of course possible that an assistant or an instructor puts the lower body assisting device on the subject; the same applies to the below) places a lengthwise end of a main body unit 10R1 of the right leg strap 10R on the front of the right thigh, presses the main body unit 10R1 gently against the right thigh with one hand to temporarily fix the position, and takes the lengthwise end of a securing unit 10R2 to the back of the right leg through between both legs, and to the front of the right thigh around the right leg. At this time, the subject applies tension to the securing unit 10R2 such that it is stretched to some extent. Then, the subject removably secure a securing unit securing member 10R21 located at the end of the reverse side of the securing unit 10R2 to a main body securing member 10R11 located in a particular range of the main body unit 10R1 on the front side at the end thereof. By securing two securing units 10R2 in this way, the right leg strap 10R is wrapped around and secured to the right thigh of the subject.

Since the entire right leg strap 10R is appropriately tensioned when the right leg strap 10R is wrapped around the right thigh, the right leg strap 10R worn around the right thigh exerts, on the right thigh muscles, a force making the right thigh muscles rotate internally.

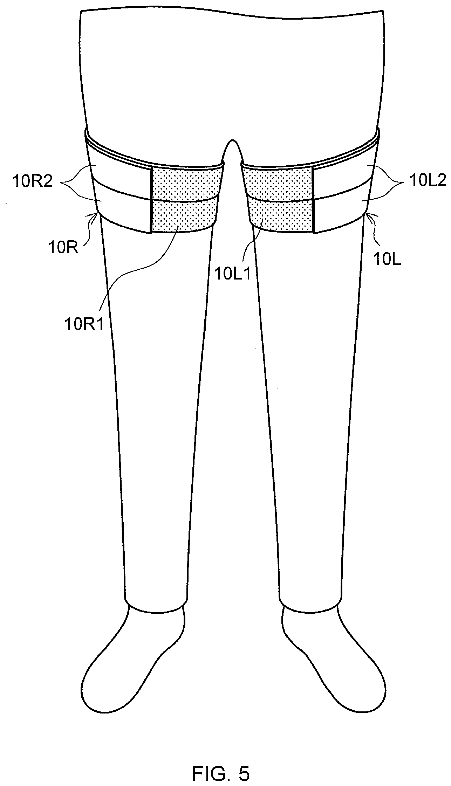

Likewise, the left leg strap 10L is secured to the left thigh of the subject at or near the top thereof. Then, the left leg strap 10L exerts, on the left thigh muscles, a force making the left thigh muscles rotate internally. Fig. 5 shows a state where the right leg strap 10R and the left leg strap 10L are worn around the respective thighs of the subject. The main body unit 10R1 of the right leg strap 10R and the main body unit 10L1 of the left leg strap 10L are both located on the back of the respective thighs.

Although it is omitted in Fig. 5, in this state, the strap front securing member 10R22 and the strap front securing members 10L22 are exposed on the back of the respective thighs.

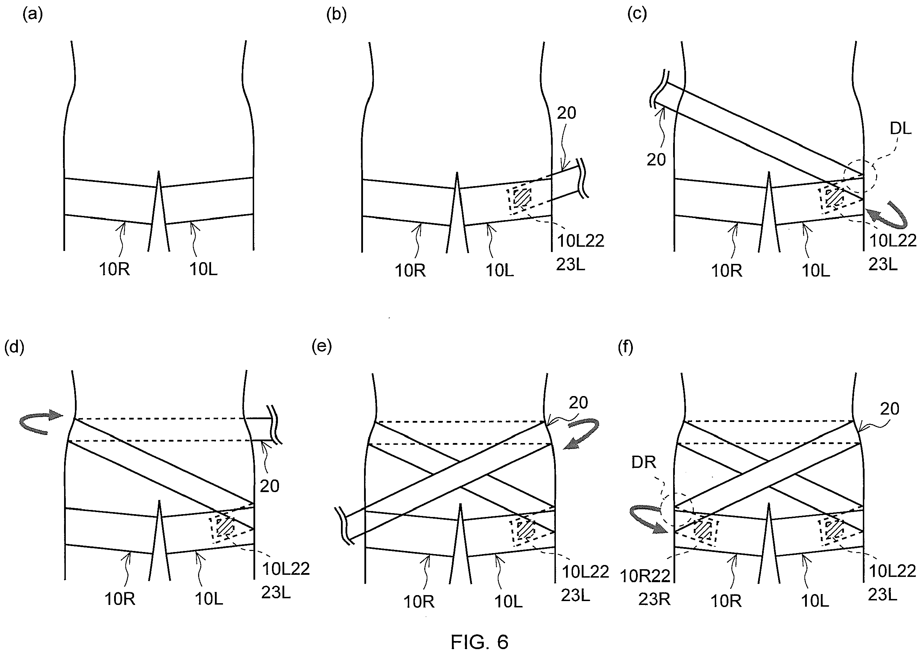

Next, the waist/hip strap 20 is secured to the right leg strap 10R and the left leg strap 10L. A way of this is shown in Fig. 6. It is noted that Fig. 6 is a view of a subject seen from the front and details of the right leg strap 10R, the left leg strap 10L, and the waist/hip strap 20 are omitted in this figure. However, the strap front securing member 10R22 and the waist/hip strap reverse right securing member 23R, and the strap front securing members 10L22 and the waist/hip strap reverse left securing member 23L, when removably secured to each other, are illustrated.

In securing the waist/hip strap 20 to the right leg strap 10R and the left leg strap 10L, in this embodiment, the subject secures the right leg strap 10R and the left leg strap 10L to the respective thighs, as shown in Fig. 6 (a) .

Next, as shown in (b) in the same figure, the subject removably secures the waist/hip strap reverse left securing member 23L of the waist/hip strap 20 to the strap front securing members 10L22 of the left leg strap 10L. In other words, the proximal end of the waist/hip strap 20 is secured to the back of the left thigh.

Next, as shown in (c) in the same figure, the subject folds the flap of the waist/hip strap 20 along the left side of the hip toward the front and brings up the flap to a front upper portion of the right ilium. Care should be taken to ensure that the waist/hip strap 20 passes near (or just over) the greater trochanter DL on the left side of the waist. The greater trochanter is a projection on the proximal end of the femur.

Next, as shown in (d) in the same figure, the subject folds the flap of the waist/hip strap 20 back in toward the ilium on the right side of the waist almost horizontally, going around the left side of the waist.

Next, as shown in (e) in the same figure, the subject folds the flap of the waist/hip strap 20 toward the front along the left side of the waist and takes it to a position near the right leg strap 10R on the front side of the body.

Next, as shown in (f) in the same figure, the subject folds the flap of the waist/hip strap 20 back in behind the right thigh and removably secures the waist/hip strap reverse right securing member 23R of the waist/hip strap 20 to the strap front securing member 10R22 of the right leg strap 10R. Care should be taken to ensure that the waist/hip strap 20 passes near (or just over) the greater trochanter DR on the right side of the waist.

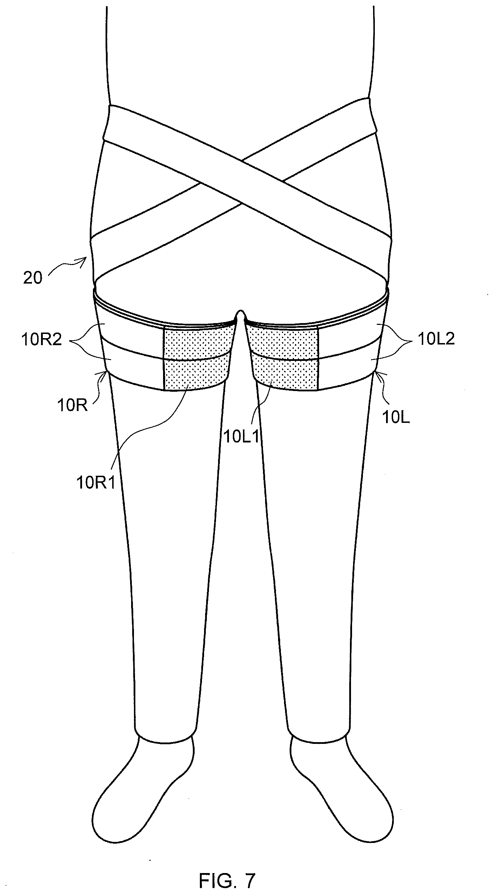

In this way, the waist/hip strap 20 is secured to the right leg strap 10R and the left leg strap 10L. Figs. 7, 8, and 9 show front, side, and back views, respectively, of the subject with the waist/hip strap 20 secured to the right leg strap 10R and the left leg strap 10L. In this state, the waist/hip strap 20 is appropriately tensioned. Consequently, a force in the counter-clockwise direction when seen from the above is exerted on the right leg strap 10R from the waist/hip strap 20, and a force in the clockwise direction when seen from the above is exerted on the left leg strap 10L from the waist/hip strap 20. These forces are for internally rotate both right and left leg straps 10R and 10L.

In securing the waist/hip strap 20 to the right leg strap 10R and the left leg strap 10L, the waist/hip strap 20 can be guided along a path in the direction reverse to the one mentioned above.

The subject can put on the right leg strap 10R, the left leg strap 10L, and the waist/hip strap 20 without using the upper body strap 30.

In this state, the right thigh muscles is internally rotated by the right leg strap 10R and the left thigh muscles are internally rotated by the left leg strap 10L. These internal rotations can alleviate the lower back pain or the issues with bowed legs of the subject. Furthermore, it is easier for the subject to move the legs forward during walking, more specifically, when moving the legs forward in a step-by-step movement. In contrast, each of the right leg strap 10R and the left leg strap 10L exerts a force on the thigh muscles against the motion when the leg moves backward in the step-by-step movement or when the retracted leg kicks the ground in a backward direction. Consequently, although the subject can walk naturally, he/she unintentionally performs thigh muscle strength training merely by walking because of the resistance thereof. Furthermore, it is easier for the sitting subject wearing the right leg strap 10R and the left leg strap 10L to stand up.

Besides, as described above, the right leg strap 10R and the left leg strap 10L always receive, from the waist/hip strap 20, the force making them rotate internally. Accordingly, even when the subject wearing them walks or stands up, the right thigh muscles internally rotated by the right leg strap 10R can sustain that internally rotated state well and the same applies to the left thigh muscles internally rotated by the left leg strap 10L.

Since the waist/hip strap 20 presses the greater trochanters of the subject toward the midline of the body, the body of the subject stabilizes while walking.

The subject can stay for 20 minutes or more with the right leg strap 10R, the left leg strap 10L and the waist/hip strap 20 worn, or perform light exercise such as walking. When the right leg strap 10R, the left leg strap 10L, and the waist/hip strap 20 are not so much tensioned, the subject can put in, for example, 2-3 hours doing daily works with the straps and belt worn, through there are individual differences.

Next, an example where the upper body strap 30 is used in addition to the right leg strap 10R, the left leg strap 10L, and the waist/hip strap 20 is described.

The upper body strap 30 is indirectly secured to the outsides of the right leg strap 10R and the left leg strap 10L when it is removably secured to the waist/hip strap 20. The upper body strap 30 can be directly secured to the outsides of the right leg strap 10R, the left leg strap 10L by providing an appropriate securing member on the outsides of the right leg strap 10R and the left leg strap 10L. In this case, there is an option to use only the right leg strap 10R, the left leg strap 10L, and the upper body strap 30 and not to use the waist/hip strap 20.

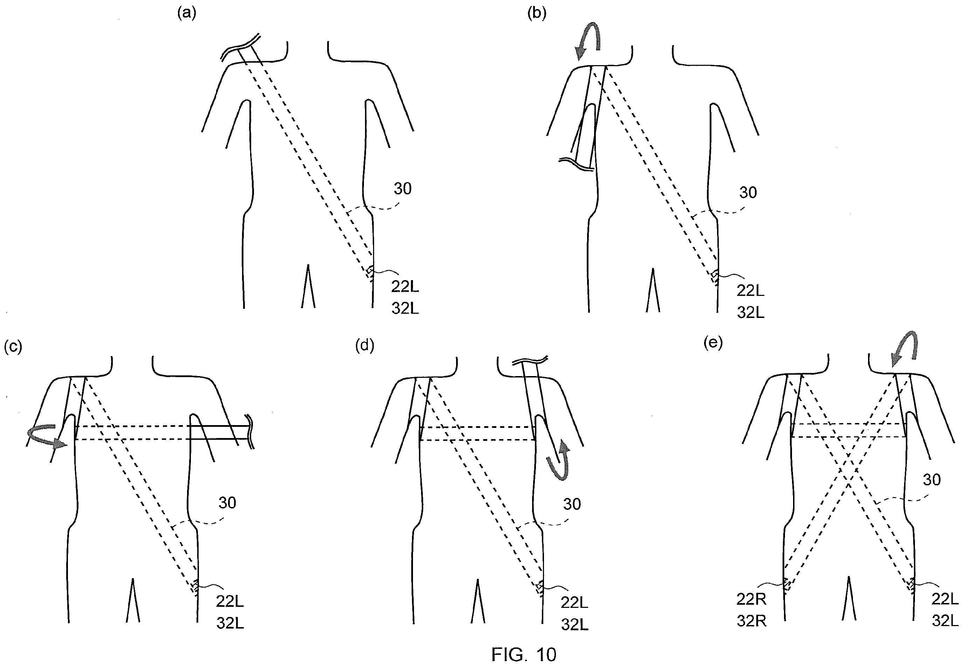

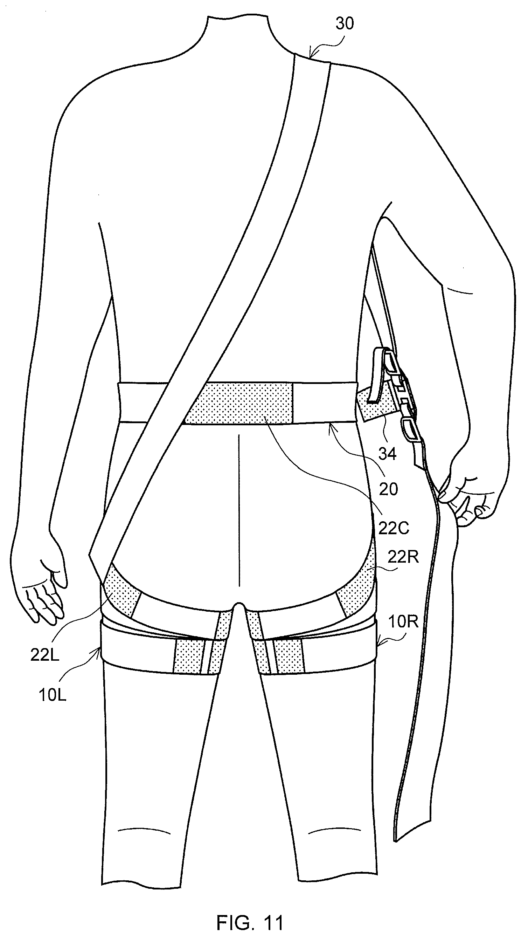

A process of securing the upper body strap 30 to the waist/hip strap 20 is shown in Figs. 10 and 11. Fig. 10 is a view of a subject seen from the front and details of the right leg strap 10R, the left leg strap 10L, and the waist/hip strap 20 are omitted in this figure for the purpose of simplifying it. However, the waist/hip strap front right securing member 22R and the upper body strap right securing member 32R, and the waist/hip strap front left securing member 22L and the upper body strap left securing member 32L, which are removably secured to each other, are illustrated.

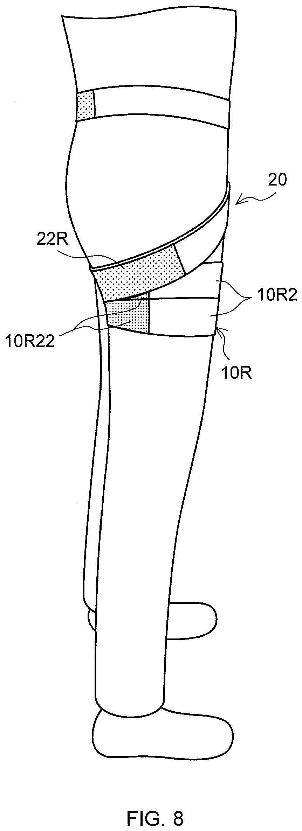

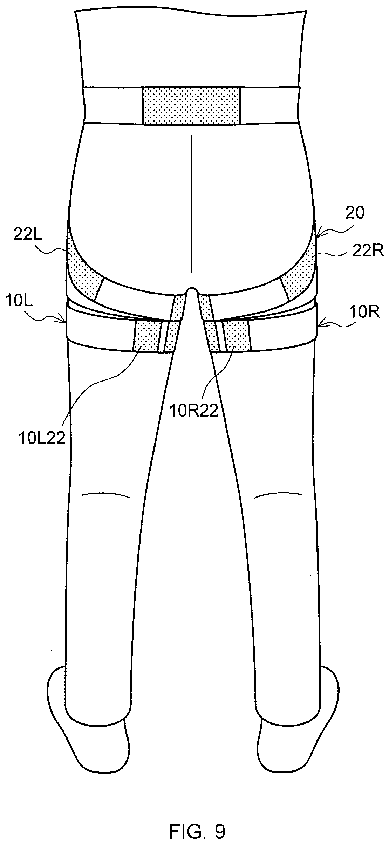

It is apparent from, for example, Figs. 8 and 9 that the waist/hip strap front right securing member 22R and the waist/hip strap front left securing member 22L are positioned on the outsides of the respective thighs.

In securing the upper body strap 30 to the waist/hip strap 20, in this embodiment, the subject first puts on the right leg strap 10R, the left leg strap 10L, and the waist/hip strap 20 as described above.

Next, as shown in Fig. 10(a), the subject removably secure the upper body strap left securing member 32L of the upper body strap 30 to the waist/hip strap front left securing member 22L of the waist/hip strap 20. Specifically, the proximal end of the upper body strap 30 is secured to the outside of the left thigh. In this embodiment, wherever the proximal end of the upper body strap 30 is secured, it comes to a position near (or just over) the left greater trochanter. The subject takes the flap of the upper body strap 30 over the right shoulder along the back side of the body of the subject.

Next, as shown in (b) in the same figure and Fig. 11, the subject folds the flap of the upper body strap 30 along the right shoulder from the back toward the front side of the body of the subject.

Next, as shown in (c) in the same figure, the subject folds the flap of the upper body strap 30 back in toward the left underarm along the right underarm of the subject, pulling the slap almost horizontally.

Next, as shown in (d) in the same figure, the subject pulls the flap of the upper body strap 30 along the left underarm to the front side of the body of the subject, and brings up the flap in front to the left shoulder of the subject.

Next, as shown in (e) in the same figure, the subject folds back the flap of the upper body strap 30 over the left shoulder and takes the flap of the upper body strap 30 along the back side of the body to the outside of the right leg of the subject. Then, the subject secures the upper body strap right securing member 32R of the upper body strap 30 to the waist/hip strap front right securing member 22R of the waist/hip strap 20. Specifically, the distal end of the upper body strap 30 is secured to the outside of the right thigh. In this embodiment, wherever the distal end of the upper body strap 30 is secured, it comes to a position near (or just over) the right greater trochanter.

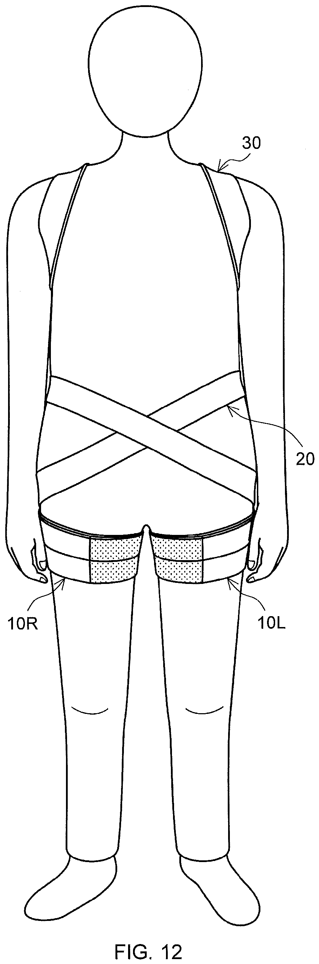

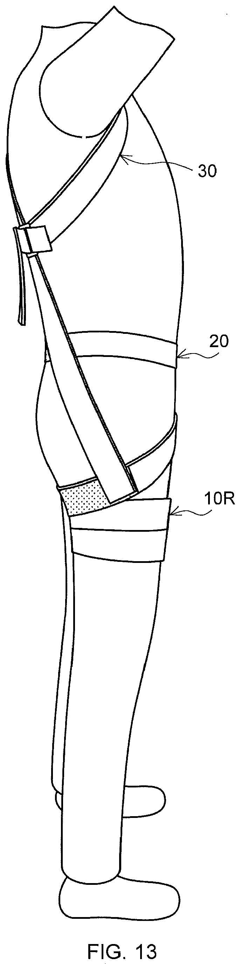

In this way, the upper body strap 30 is secured to the waist/hip strap 20 or to the right leg strap 10R and the left leg strap 10L via the waist/hip strap 20. Figs. 12, 13, and 14 show front, side, and back views, respectively, of the subject wearing the upper body strap 30 secured to the waist/hip strap 20 or to the right leg strap 10R and the left leg strap 10L via the waist/hip strap 20.

In this state, the upper body strap 30 is appropriately tensioned. The upper body strap 30 helps retract both shoulders of the subject backward and thereby allow him/her to naturally assume an upright posture with the chest puffed out. In addition, the upper body strap 30 can prevent swing and rotation of the body of the subject while walking, and thus the subject can continuously assume an upright posture, for example, even during walking. Furthermore, the upper body strap 30 assists the subject in walking.

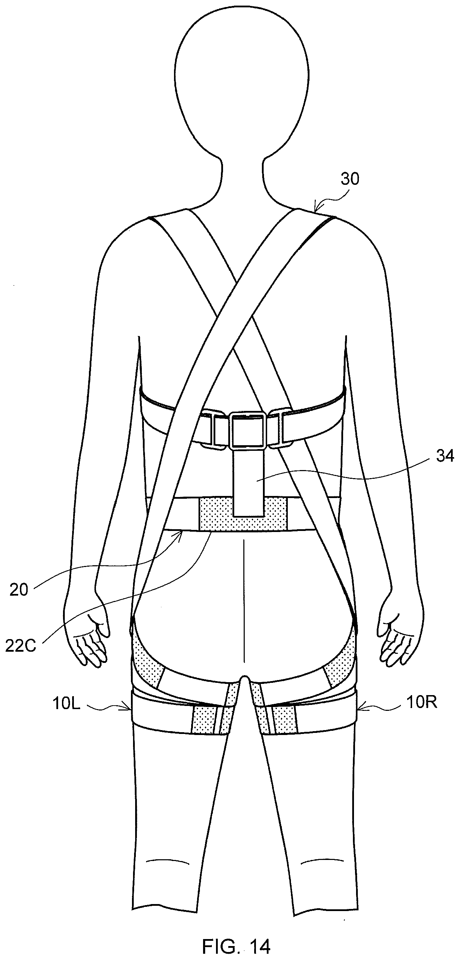

Furthermore, in securing the upper body strap 30 to the waist/hip strap 20, in particular, as shown in Fig. 14, the lower end of the connection band 34 of the upper body strap 30 which is not illustrated in Fig. 10 can be connected to the center of the almost horizontal portion of the waist/hip strap 20 that is located immediately below it. This connection is made by securing the upper body strap center securing member 32C on the reverse side of the lower end of the connection band 34 of the upper body strap 30 to the waist/hip strap front center securing member 22C. The length of the connection band 34 is slightly shorter than the distance between the almost horizontal portion of the waist/hip strap 20 and the almost horizontal portion of the upper body strap 30, both of which are in contact with the back of the subject. Therefore, when the upper body strap center securing member 32C of the upper body strap 30 is secured to the waist/hip strap front center securing member 22C of the waist/hip strap 20, the connection band 34 is tensioned. Consequently, a force making the upper body of the subject retract is exerted, which further improves the posture of the subject.

The connection band 34 in this embodiment is secured to the upper body strap 30 at the end closer to the upper body strap 30, and the end closer to the waist/hip strap 20 can be removably attached to the waist/hip strap 20. This relationship can be reversed or their ends can be adapted to be removably attached to the upper body strap 30 and the waist/hip strap 20.

With the upper body strap 30 used, the length of time that the lower body assisting device can be worn is similar to that in the cases where the upper body strap 30 is not used.

### <Modified version 1>

A lower body assisting device according to a modified version 1 is described.

The lower body assisting device of the modified version 1 is almost identical in all of its construction and how it is used to those in the first embodiment, and how it is used is completely the same.

The only difference lies in the construction of the right leg strap 10R and the left leg strap 10L included in the lower body assisting device.

As in the case of the first embodiment, the construction of the left leg strap 10 according to the modified version 1 is described for the left leg strap 10L as an example. In the modified version 1, the right leg strap 10R is in mirror symmetry to the left leg strap 10L.

The left leg strap 10L included in the lower body assisting device according to the modified version 1 has the main body unit 10L1 and the securing units 10L2 which are curved upwardly in use. Specifically, the left leg strap 10L included in the lower body assisting device according to the modified version 1 has upper and lower edges which are curved (e.g., a part of an arc or a part of an ellipse or other quadratic curve) upwardly in use. A human thigh typically gets thicker as it gets closer to the top. If the left leg strap 10L has the same width along the entire length thereof as in the case of the first embodiment, a gap may be formed between a lower portion of the left leg strap 10L and the thigh when the left leg strap 10L is wrapped around the left thigh. Then, the left leg strap 10L can fail to achieve the internal rotation of the left thigh muscles. If the aforementioned upward curve helps the left leg strap 10L fit and follow the contour of the left thigh well when the left leg strap 10L is wrapped around the left thigh, the above inconvenience can be avoided.

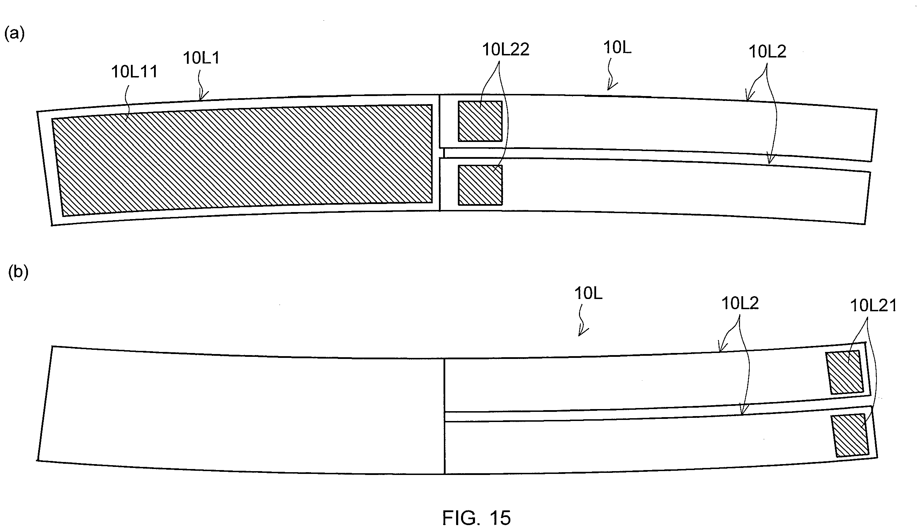

From such perspectives, it is better that the length of the upward curve along the upper edge of the left leg strap 10L is greater than the length of the upward curve along the lower edge. In this sense, it is better that the lengthwise opposite edges of the left leg strap 10L and the right leg strap are, if they are all straight, getting closer as they get closer to the bottom, as shown in Fig. 15.

### <Modified version 2>

A lower body assisting device according to a modified version 2 is described.

The lower body assisting device of the modified version 2 is almost identical in all of its construction and how it is used to those in the first embodiment.

The only difference lies in the construction of the right leg strap 10R and the left leg strap 10L included in the lower body assisting device, and a way of putting them on the right and left thighs of the subject.

As in the case of the first embodiment, the construction of the left leg strap 10 according to the modified version 2 is described for the left leg strap 10L as an example. In the modified version 2, the right leg strap 10R is in mirror symmetry to the left leg strap 10L.

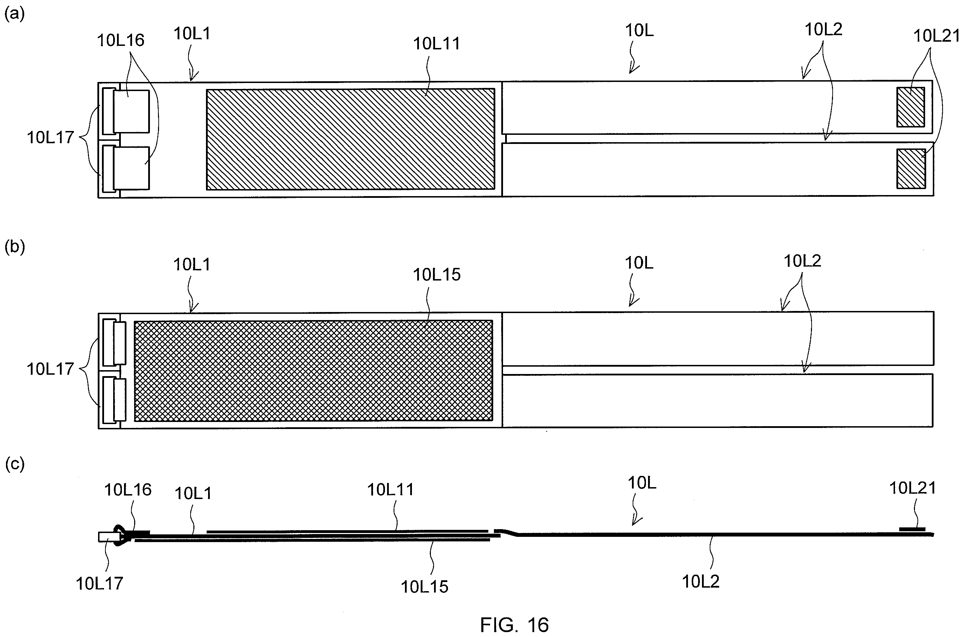

The left leg strap 10L included in the lower body assisting device according to the modified version 2 is constructed as shown in Fig. 16.

The left leg strap 10L of the modified version 2 has the main body unit 10L1 and two securing units 10L2, as in the case of the first embodiment. The width of the left leg strap 10L is similar to that in the first embodiment and the length is slightly greater than that in the first embodiment.

The length of the main body unit 10L1 in the left leg strap 10L of the modified version 2 is similar to that in the first embodiment and the length of each securing unit 10L2 is greater by about 15 cm than that in the first embodiment.

The left leg strap 10L of the modified version 2 is provided with the main body securing member 10L11 on the front side thereof. The left leg strap 10L of the modified version is provided with the securing unit securing members 10L21 that can be attached to and removed from the main body securing member 10L11, as in the case of the first embodiment. However, unlike the first embodiment, the securing unit securing members 10L21 of the modified version 2 are secured to the securing units 10L2 on the front side thereof, not the reverse side.

Furthermore, the main body unit 10L1 of the left leg strap 10L of the modified version 2 is provided with, almost over the entire reverse side, a piece of mesh sheet 10L15 which is not present in the first embodiment. The mesh sheet 10L15 is a known or widely known mesh cloth, which is used for making the left leg strap 10L breathable or making it kind to the skin when secured to the left thigh of the subject.

In addition, the left leg strap 10L of the modified version 2 does not have the strap front securing members 10L22 which are present in the first embodiment.

On one end along the length of the left leg strap 10L of the modified version 2 opposite to the end where the securing units 10L2 are attached, two buckles 10L17 are attached in such a manner that they are aligned vertically. The buckles 10L17 are formed into an elongated rectangle as a whole and have rectangular holes. The size and the shape of the holes in the two, upper and lower buckles 10L17 are such that the securing units 10L2 aligned vertically can pass through.

Although the buckles 10L17 can be secured to the main body unit 10L1 by any means, each buckle 10L17 in this embodiment is secured by stitching both ends of a piece of cloth 10L16 that is passed through the buckle 10L17 and flipped into half to the ends of the front and reverse sides of the main body unit 10L1.

The right leg strap 10R and the left leg strap 10L are different from those of the first embodiment in how it is used, particularly a way of putting them on the thigh of the subject.

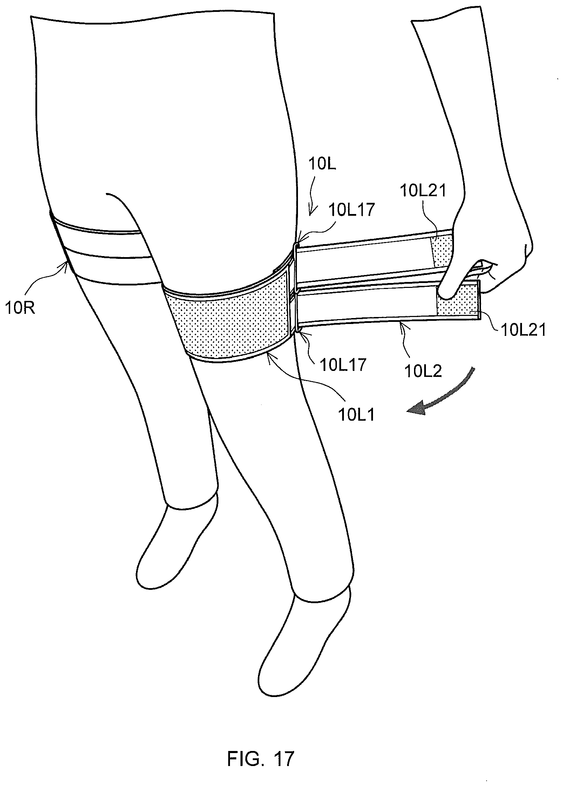

By way of example, a way of putting the left leg strap 10L on the left thigh of the subject is described. For this description, Fig. 17 is used.

In order to put the left leg strap 10L of the modified version 2 on the left thigh, the left leg strap 10L is passed through between both legs with the main body unit 10L1 placed on the back and the securing units 10L2 on the front, and the reverse side thereof facing the left thigh. In this state, while adjusting the buckles 10L17 to positions behind the outside of the left leg, the flaps of the upper and lower securing units 10L2 are passed through the respective buckles 10L17. Fig. 17 shows that state. Then, the flaps of the securing units 10L2 are pulled toward the front of the legs as indicated by the arrow in the figure, and the securing unit securing members 10L21 at the ends of the respective securing units 10L2 are removably secured to the main body securing member 10L11. In this case, when the left leg strap 10L is secured to the left thigh, a force is exerted in a direction from behind the outside of the left leg to the front inwardly (i.e., the securing units 10L2 are pulled), whereby the left thigh muscles rotate internally.

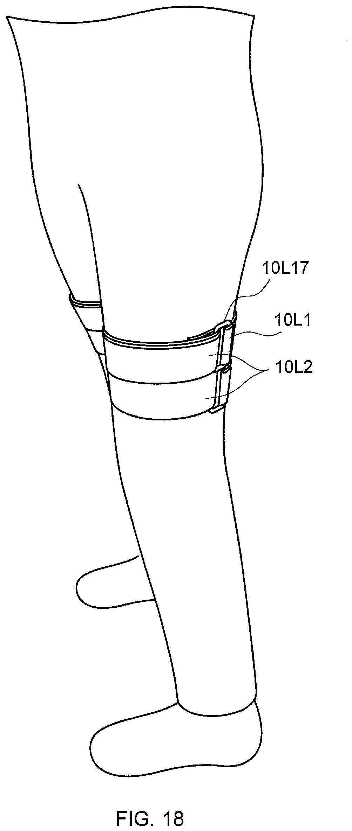

Fig. 18 shows the left leg strap 10L secured to the left thigh.

In summary, unlike the first embodiment, in the left leg strap 10L of the modified version 2, the securing unit 10L2 passed through the buckle 10L17 is flipped at the buckle 10L17 and then secured to the main body securing member 10L11. Because of this flip, unlike the first embodiment, in the left leg strap 10L of the modified version 2, the securing unit securing member 10L21 is secured to the front side of the securing unit 10L2.

After that, the method of using the lower body assisting device according to the modified version 2 is similar to that in the first embodiment. Specifically, as in the first embodiment, the lower body assisting device according to the modified version 2 can be used in combination with the waist/hip strap 20 and the upper body strap 30.

It is noted that the left leg strap 10L of the lower body assisting device according to the modified version 2 does not have the strap front securing members 10L22 which are used to connect the waist/hip strap 20 and the left leg strap 10L. However, in the left leg strap 10L of the modified version 2, even when the securing unit securing members 10L21 of the securing units 10L2 are attached to the main body securing member 10L11 covering almost entire surface of the front side of the main body unit 10L1, a portion of the main body securing member 10L11 closer to the securing units 10L2 is exposed without being covered with the securing units 10L2. The exposed portion of the main body securing member 10L11 is located immediately behind the buckles 10L17, i.e., on the back of the left leg. By using the main body securing member 10L11 exposing immediately behind the buckles 10L17 in place of the strap front securing members 10L22 in the first embodiment, the removable attachment between the waist/hip strap 20 and the left leg strap 10L can also be achieved in the lower body assisting device of the modified version 2.

The removable attachment between the waist/hip strap 20 and the right leg strap 10R can also be achieved in a similar manner.

The removable attachment of the upper body strap 30 to the waist/hip strap 20 is identical to that of the first embodiment.

### <<Second embodiment>>

A lower body assisting device of a second embodiment is described.

The lower body assisting device of the second embodiment is, in one word, a device in which the right leg strap 10R, the waist/hip strap 20, and the left leg strap 10L in the lower body assisting device of the first embodiment are united in this order.

The lower body assisting device of the second embodiment is, as described above, basically a united substance.

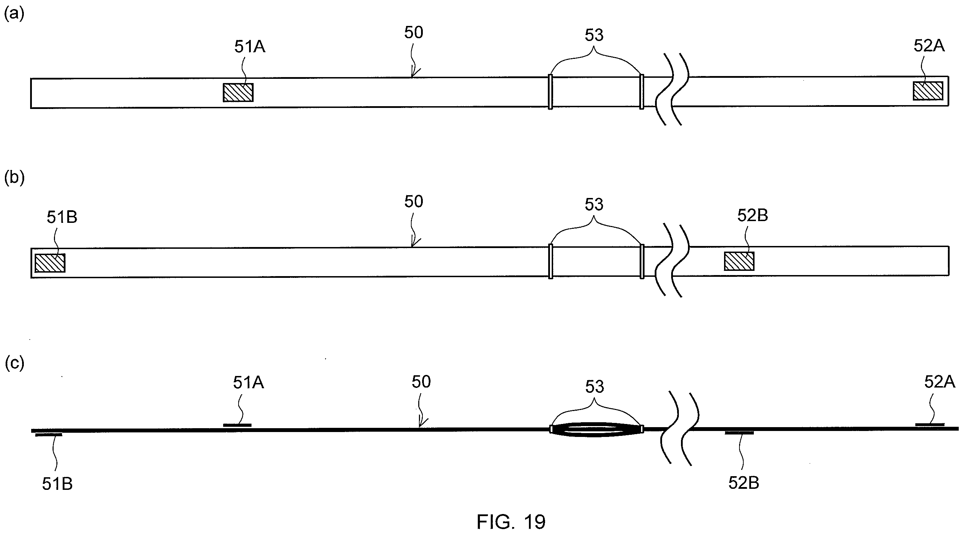

The lower body assisting device has, as shown in Fig. 19, a contiguous main body 50. The main body 50 is, but not limited to, a strip-shaped substance with the same width along the entire length thereof. In other words, the main body 50 has a considerably long rectangular shape. The length is determined such that a method of using the lower body assisting device as described later can be performed and is, in many cases, around 200-300 cm, though depending on the frame of the subject. The width thereof is, but not limited to about 5-15 cm. The reason the width is determined in this range the same as the reason the width of the left leg strap 10L is determined in the first embodiment.

The main body 50 of the lower body assisting device is provided with a pair of securing members 51A, 51B on the front and reverse sides, respectively, of the main body 50 at a position closer to the edge of one end thereof, and a pair of securing members 52A, 52B on the front and reverse sides, respectively, of the main body 50 at a position closer to the edge of the other end thereof.

The pair of securing member 51A and securing member 51B can be removably secured to each other. The pair of securing member 52A and securing member 52B can be removably secured to each other.

One of the securing members 51A and 52A on the front side is provided near the lengthwise end of the main body 50 and the other is provided at a position closer, by a distance slightly shorter than the circumference of a thigh of a subject, to the center along the length of the main body 50 than the lengthwise end of the main body 50.

One of the securing members 51B and 52B on the reverse side is provided near the lengthwise end of the main body 50 and the other is provided at a position closer, by a distance slightly shorter than the circumference of a thigh of a subject, to the center along the length of the main body 50 than the lengthwise end of the main body 50. It is noted that the securing members 51B and 52B are located slightly inward on the main body 50 when their respective mating securing members 51A and 52A are located near the lengthwise end of the main body 50, whereas they are located near the end of the main body 50 when their respective mating securing members 51A and 52A are located slightly inward along the length of the main body 50.

Near the center of the main body 50, length adjustment members 53 are provided for adjusting the lengthwise dimension of the main body 50 are provided. The construction and function of the length adjustment members 53 are identical to those of length adjustment members 33 in the first embodiment.

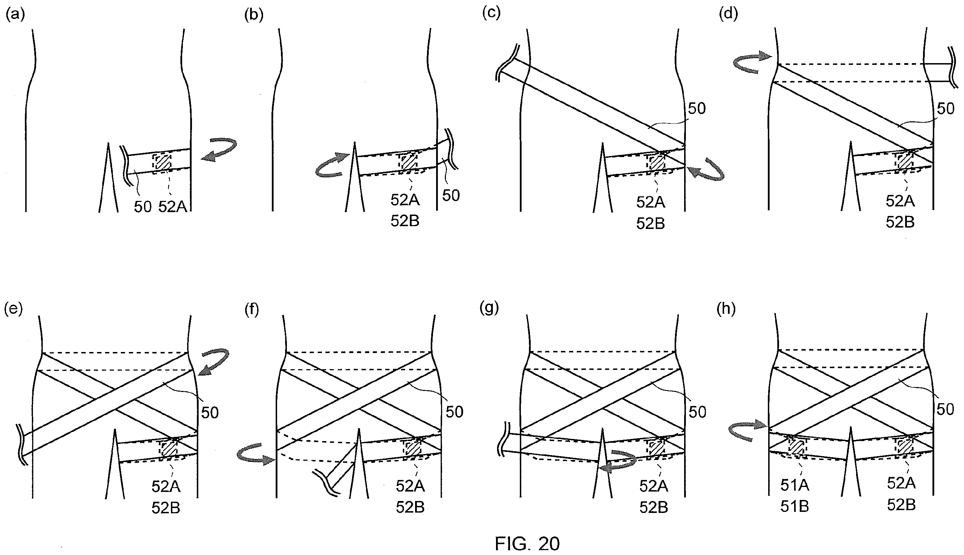

Next, a method of using the lower body assisting device of the second embodiment and effects thereof are described. For this description, Fig. 20 is used. In the figure, the subject is facing front. In Fig. 20, only the main body 50, the securing members 51A and 51B when secured to each other, and the securing members 52A and 52B when secured to each other are shown.

To use this lower body assisting device, as shown in Fig. 20(a), with the securing member 52A on the front side at one end being exposed outside, the end with the securing member 52A is placed on the back of one thigh (which is the left thigh in this embodiment, but not limited thereto) of the subject. The flap of the main body 50 is taken toward the front around the left leg of the subject.

Next, as shown in (b) in the same figure, the flap of the main body 50 is passed through between both legs and taken to the back again. The securing member 52B on the reverse side of the main body 50 is removably secured to the securing member 52A. In this state, the main body 50 is secured around the left thigh. At this time, the portion of the main body 50 spanning from the securing member 52A to the securing member 52B is wrapped around the left thigh in a direction of internal rotation, so that a force making the left thigh muscles rotate internally is exerted on the muscles. The portion of the main body 50 spanning from the securing member 52A to the securing member 52B functions like the left leg strap 10L in the first embodiment.

Subsequent processes of handling the main body 50 are similar to those used to put on the waist/hip strap 20 in the first embodiment.

Next, as shown in (c) in the same figure, the subject folds the flap of the main body 50 along the left side of the hip toward the front and brings up the flap to a front upper portion of the right ilium. Care should be taken to ensure that the main body 50 passes near (or just over) the greater trochanter on the left side of the waist.

Next, as shown in (d) in the same figure, the subject folds the flap of the main body 50 back in toward the left side of the waist almost horizontally, going around the right side of the waist.

Next, as shown in (e) in the same figure, the subject folds the flap of the main body 50 toward the front along the left side of the waist and takes it to a position near the outside of the right thigh on the front side of the body.

Next, as shown in (f) in the same figure, the subject folds the flap of the main body 50 back in behind the right thigh and brings it to a groin position almost horizontally along the back of the right thigh. Care should be taken to ensure that the securing member 51A is located slightly inward on the back of the right thigh. Also, care should be taken to ensure that the main body 50 passes near (or just over) the greater trochanter on the right side of the waist.

Next, as shown in (g) in the same figure, the subject brings the flap of the main body 50 from the groin to the front of the body and further to the outer front side of the right thigh.

Next, as shown in (h) in the same figure, the subject folds the flap of the main body 50 around the outside of the right thigh to the back of the right thigh. Then, the subject removably secures the securing member 51B on the reverse side of the flap to the securing member 51A. As a result, the portion of the main body 50 spanning from the securing member 51A to the securing member 51B makes a loop encircling the right thigh. This portion is similar to the right leg strap 10R in the first embodiment. However, the portion of the main body 50 spanning from the securing member 51A to the securing member 51B is wrapped around the right thigh in a direction of external rotation of the muscles and does not function like the right leg strap 10R in the first embodiment unless the subject does something further. The subject then holds the portion of the main body 50 spanning from the securing member 51A to the securing member 51B and forcefully rotates it. Consequently, the portion of the main body 50 spanning from the securing member 51A to the securing member 51B rotates the right thigh muscles internally and thus functions like the right leg strap 10R in the first embodiment.

The function of the lower body assisting device in the second embodiment is identical to the function achieved by combining the right leg strap 10R and the left leg strap 10L with the waist/hip strap 20 in the first embodiment.

On a portion of the main body 50 that functions as the right leg strap 10R in the first embodiment (i.e., the portion encircling the right thigh) and a portion thereof that functions as the left leg strap 10L in the first embodiment (i.e., the portion encircling the left thigh), a force making these portions rotate internally is exerted by a portion of the main body 50 that crosses on the front side of the waist and then is brought behind the thigh. This force is equivalent to the force that the waist/hip strap 20 in the first embodiment exerts on the right leg strap 10R and the left leg strap 10L.

### Denotation of symbols

- 10R: right leg strap
- 10R1: main body unit
- 10R11: main body securing member
- 10R2: securing unit
- 10R21: securing unit securing member
- 10R22: strap front securing member
- 10L: left leg strap
- 10L1: main body unit
- 10L11: main body securing member
- 10L2: securing unit
- 10L21: securing unit securing member
- 10L22: strap front securing member
- 20: waist/hip strap
- 21: waist/hip strap main body
- 22R: waist/hip strap front right securing member
- 22L: waist/hip strap front left securing member
- 22C: waist/hip strap front center securing member
- 23R: waist/hip strap reverse right securing member
- 23L: waist/hip strap reverse left securing member
- 30: upper body strap
- 31: upper body strap main body
- 32R: upper body strap right securing member
- 32L: upper body strap left securing member
- 32C: upper body strap center securing member
- 34: connection band
- 50: main body
- 51A: securing member
- 51B: securing member
- 52A: securing member
- 52B: securing member

## Claims

1. A lower body assisting device comprising:
a right leg strap being a strip-shaped substance for exerting, on the right thigh of a subject, a force making the right thigh muscles rotate internally by wrapping the right leg strap around the right thigh at a predetermined compression pressure in use; and
a left leg strap being a strip-shaped substance for exerting, on the left thigh of the subject, a force making the left thigh muscles rotate internally by wrapping the left leg strap around the left thigh at a predetermined compression pressure in use.

2. The lower body assisting device according to Claim 1, further comprising an anti-rotation member for preventing the right leg strap wrapped around the right thigh from rotating in a direction of external rotation of the muscles of the right thigh and preventing the left leg strap wrapped around the left thigh from rotating in a direction of external rotation of the muscles of the left thigh, the anti-rotation member being secured to the body of the subject.

3. The lower body assisting device according to Claim 2, wherein the anti-rotation member is a strip-shaped substance.

4. The lower body assisting device according to Claim 2, wherein the anti-rotation member is configured to link the right leg strap and the left leg strap to each other.

5. The lower body assisting device according to any one of Claims 2 to 4, wherein the anti-rotation member can be attached to and removed from the right leg strap and the left leg strap.

6. The lower body assisting device according to Claim 2, wherein the right leg strap, the anti-rotation member, and the left leg strap are linked in this order and compose a unitary strip-shaped substance.

7. The lower body assisting device according to Claim 3, wherein the anti-rotation member is a strip-shaped substance having a length that allows the anti-rotation member to connect the back of the right leg strap worn on the right leg of the subject and the back of the left leg strap worn on the left leg of the subject with each other along a path:
from the back of the right leg strap to the left ilium of the subject, going around the outside of the right leg strap and along the front side of the body of the subject;
from the left ilium, folding back in toward the right ilium of the subject, going along the back side of the body of the subject; and
from the right ilium to the back of the left leg, going along the front side of the body of the subject and around the outside of the left leg strap, or vise versa.

8. The lower body assisting device according to Claim 1, wherein a substantial half of a longitudinal length of a part of each of the right leg strap and the left leg strap is less easily stretchable, the longitudinal length of the part of each of the right leg strap and the left leg strap corresponding to a circumference of the respective right and left thighs.

9. The lower body assisting device according to Claim 1, wherein upper and lower edges of each of the right leg strap and the left leg strap in use are curved upwardly and the upper edge is longer than the lower edge, whereby the right leg strap and the left leg strap fit on the respective legs of the subject when worn thereon.

10. The lower body assisting device according to Claim 1 or 2, further comprising an upper body strap, the upper body strap being a strip-shaped substance having a length that allows the upper body strap to connect the outside of the right leg strap worn on the right leg of the subject and the outside of the left leg strap worn on the left leg of the subject with each other along a path:
from the outside of the right leg strap to the top of the left shoulder of the subject along the back side of the body of the subject;
from the top of the left shoulder to the left underarm of the subject along the front side of the body of the subject;
from the left underarm to the right underarm of the subject along the back side of the body of the subject;
from the right underarm to the top of the right shoulder of the subject along the front side of the body of the subject; and
from the top of the right shoulder to the outside of the left leg strap along the back side of the body of the subject, or vise versa, wherein
both ends of the upper body strap can be attached to and removed from the respective outsides of the right leg strap and the left leg strap.

11. The lower body assisting device according to Claim 10, wherein the upper body strap is stretchable lengthwise.

12. The lower body assisting device according to Claim 7, further comprising an upper body strap, the upper body strap being a strip-shaped substance having a length that allows the upper body strap to connect the outside of the right leg strap worn on the right leg of the subject and the outside of the left leg strap worn on the left leg of the subject with each other along a path:
from the outside of the right leg strap to the top of the left shoulder of the subject along the back side of the body of the subject;
from the top of the left shoulder to the left underarm of the subject along the front side of the body of the subject;
from the left underarm to the right underarm of the subject along the back side of the body of the subject;
from the right underarm to the top of the right shoulder of the subject along the front side of the body of the subject; and
from the top of the right shoulder to the outside of the left leg strap along the back side of the body of the subject, or vise versa; and
a vertical connection member, the vertical connection member being configured so as to connect a substantial center of the portion of the upper body strap running from the left underarm to the right underarm along the back side of the body of the subject and a substantial center of the portion of the anti-rotation member running from the left ilium to the right ilium along the back side of the body of the subject.

13. A lower body assisting method comprising the steps of:
wrapping a right leg strap, being a strip-shaped substance, around the right thigh of a subject in such a manner that the right leg strap exerts, on the right thigh, a force making the right thigh muscles rotate internally; and
wrapping a left leg strap, being a strip-shaped substance, around the left thigh of the subject in such a manner that the left leg strap exerts, on the left thigh, a force making the left thigh muscles rotate internally.
